# FASCICULE DE BREVET EUROPEEN

(11) **EP 3 184 609 B1**
(45) Date de publication et mention de la délivrance du brevet: **01.08.2018**
(21) Numéro de dépôt: 16306698.8
(22) Date de dépôt: 15.12.2016
(51) Int. Cl.: C10G 50/00, C10G 7/00, C10G 7/02, C10G 57/02

(54) **PROCEDE DE FRACTIONNEMENT POUR UN PROCEDE D'OLIGOMERISATION D'OLEFINES LEGERES**
FRAKTIONIERUNGSVERFAHREN FÜR EIN OLIGOMERISATIONSVERFAHREN VON LEICHTOLEFINEN
FRACTIONATION METHOD FOR A METHOD FOR OLIGOMERISING LIGHT OLEFINS

(30) Priorité: 22.12.2015 FR 1563036
(43) Date de publication de la demande: 28.06.2017
(73) Titulaire: AXENS, 92508 Rueil-Malmaison Cedex (FR)
(72) Inventeur: PREVOST, Isabelle, 92500 RUEIL MALMAISON (FR); PIGOURIER, Jérôme, 92190 MEUDON (FR)
(74) Mandataire: Schmitt, Nicolas A.J.

(56) Documents cités:
- FR-A1- 2 968 010
- US-A- 4 504 691
- US-A1- 2014 134 064

## Description

### DOMAINE DE L'INVENTION

L'invention se situe dans le domaine de la production d'oligomères à partir d'oléfines légères, et plus particulièrement les oligomères ayant plus de 8 atomes de carbone présentant des points d'ébullition situés dans la gamme des essences, du kérosène et du gasoil. En particulier, l'invention concerne l'étape de séparation des produits obtenus au cours d'un procédé d'oligomérisation.

### ART ANTÉRIEUR

Les réactions d'oligomérisation conduisent à différents produits présentant des points d'ébullition différents, ainsi, en aval de la section d'oligomérisation, une section de fractionnement est nécessaire pour isoler les produits recherchés tels que le les gaz de pétrole liquéfiés (GPL), les essences et les distillats moyens.

De manière générale la section de fractionnement d'un procédé d'oligomérisation permettant d'obtenir des GPL, des essences et des distillats moyens met en jeu deux colonnes de fractionnement. L'effluent de la section d'oligomérisation est envoyé dans une première distillation, le débutaniseur, qui permet d'éliminer les composants les plus légers principalement les hydrocarbures contenant 4 atomes de carbone ou moins, afin de produire une coupe essence légère dont la tension de vapeur est comprise entre 0,005 et 0,09 MPa. Ainsi, le débutaniseur produit en tête un flux riche en hydrocarbures contenant 4 atomes de carbone ou moins qui est envoyé à un stockage de gaz de pétrole liquéfié (GPL), et en fond un mélange d'essence et de distillat qui est ensuite fractionné dans une deuxième distillation, le séparateur (splitter selon la terminologie anglo-saxone), afin de produire l'essence et le distillat aux spécifications requises pour la raffinerie. Afin de minimiser les consommations énergétiques de l'étape de fractionnement, les pressions opératoires des deux colonnes sont minimisées sans toutefois diminuer leurs températures de condensation au-dessous des conditions de refroidissement réalisables par les utilités froides disponibles. Ainsi les températures des condenseurs des colonnes se situent typiquement entre environ 30°C et environ 50°C, et les pressions sont de l'ordre de 0,6 à 0,7 MPa pour le débutaniseur et généralement de l'ordre de la pression atmosphérique (0,1 MPa) pour le séparateur.

Le brevet US 4 720 600 décrit un procédé d'oligomérisation d'oléfines légères dans lequel des oléfines comportant un nombre de carbone compris entre 3 et 6 sont oligomérisées dans une section réactionnelle en présence d'un catalyseur zéolitique. L'effluent obtenu est fractionné au moyen de 3 colonnes afin d'obtenir un distillat, qui constitue le produit recherché, une fraction légère et une fraction lourde qui sont au moins en partie recyclées dans la section réactionnelle d'oligomérisation. Ledit distillat, situé dans le domaine du kérosène, comporte en particulier les composés présentant un point d'ébullition compris entre 165°C et 290°C.

La demande de brevet US 2014/0134064 décrit un dispositif de fractionnement permettant de valoriser un oligomérat selon deux variantes de section de fractionnement contenant une succession de 3 colonnes de distillation pour obtenir 3 produits principaux et un flux d'hydrocarbures en C5, en aval de la section réactionnelle d'oligomérisation. Selon une première variante, ladite section de fractionnement comprend une première colonne de fractionnement (débutaniseur) séparant les hydrocarbures contenant de 1 à 4 atomes de carbone et les hydrocarbures contenant au moins 5 atomes de carbone. La fraction comprenant les hydrocarbures contenant au moins 5 atomes de carbone est introduite dans une seconde colonne de fractionnement (dépentaniseur) qui permet de récupérer en tête les hydrocarbures contenant 5 atomes de carbone qui peuvent être recyclés à la section réactionnelle et en fond de colonne les hydrocarbures contenant au moins 6 atomes de carbone dont une partie peut être valorisée comme base essence et une seconde partie envoyée vers une troisième colonne de fractionnement (colonne de récupération de distillats moyens) qui permet de séparer une coupe comprenant les hydrocarbures contenant de 6 à 9 atomes de carbone et une coupe diesel. Selon une deuxième variante, la deuxième colonne de fractionnement (dépentaniseur) est remplacée par un strippeur adjacent au débutaniseur (side-stripper selon la terminologie anglo-saxonne). Ce strippeur est alimenté par un soutirage latéral placé sur le débutaniseur et suffisamment en tête de la colonne pour pouvoir prélever une coupe d'hydrocarbures contenant 5 atomes de carbone. Une partie de ce soutirage latéral peut être recyclé au réacteur d'oligomérisation de façon à maintenir la zone réactionnelle d'oligomérisation en phase liquide, à contrôler l'exothermicité et à maximiser le rendement en essence, une seconde partie est envoyée vers le strippeur pour séparer les hydrocarbures contenant 4 atomes de carbone ou moins qui sont renvoyés au débutaniseur et les hydrocarbures contenant 5 atomes de carbone.

Le fractionnement de l'oligomérat requis pour séparer les trois produits désirés que sont le gaz de pétrole liquéfié, les essences et les distillats moyens est une opération couteuse qui représente typiquement plus de 70% de l'énergie totale consommée dans l'unité d'oligomérisation. La consommation d'utilités chaudes totale de la section de fractionnement correspond à la somme des services, duties selon la terminologie anglo-saxonne, de rebouillage des distillations. L'oligomérat contient des molécules à haut nombre de carbone, les températures de rebouillage des colonnes sont donc en général supérieures à 140°C pour le débutaniseur (première colonne) et supérieure à 200 °C pour la deuxième colonne de séparation essence/distillats moyens (séparateur). Ces températures de rebouillage dépendent de la composition de l'oligomérat, ainsi que du choix des pressions opératoires. De plus, il y a très peu voire pas de fluides chauds disponibles dans l'unité avec une énergie thermique suffisante pour permettre une intégration thermique.

Il existe donc un réel besoin de développement de section de fractionnement limitant la consommation énergétique.

De manière surprenante, l'intégration d'une colonne de préfractionnement a permis à la demanderesse de diminuer grandement les besoins énergétiques d'un procédé de fractionnement d'un oligomérat.

### DEFINITIONS

On entend par fluide chaud, un fluide du procédé subissant un refroidissement.

On entend par fluide froid, un fluide du procédé subissant un réchauffement.

On entend par utilité chaude, un fluide extérieur au procédé subissant un refroidissement pour apporter de la chaleur à un fluide froid.

On entend par utilité froide, un fluide extérieur au procédé subissant un réchauffement pour soutirer de la chaleur à un fluide chaud.

Ainsi, la consommation d'utilités chaude d'un procédé correspond à la quantité totale de chaleur fournie par une ou plusieurs utilités chaudes audit procédé.

On entend par service, duty selon la terminologie anglo-saxonne, la quantité d'énergie thermique transférée entre deux fluides.

Ainsi le service de rebouillage correspond à la quantité d'énergie thermique apportée dans une colonne de distillation et le service de condensation correspond à la quantité d'énergie thermique évacuée d'une colonne de distillation.

### OBJET DE L'INVENTION

L'invention concerne donc un schéma de fractionnement pour un procédé d'oligomérisation d'oléfines légères comprenant un préfractionnement de l'effluent de la section réactionnelle.

L'invention concerne plus particulièrement un procédé d'oligomérisation d'oléfines légères dans lequel l'effluent de la section d'oligomérisation est envoyé dans un préfractionnateur qui conduit à au moins une fraction de tête contenant un mélange de gaz de pétrole liquéfié et d'essence légère et une fraction de fond contenant un mélange d'essence lourde et de distillat moyen, ladite fraction de tête étant envoyée dans un débutaniseur qui conduit à au moins une coupe gaz de pétrole liquéfié et une coupe essence légère, ladite fraction de fond et au moins une partie de ladite coupe essence légère étant envoyée dans un séparateur permettant d'obtenir au moins une fraction gazeuse, une fraction essence et une fraction gazole.

Un avantage de la présente invention est de réduire significativement la consommation d'utilités chaudes sans impacter les quantités et qualités de GPL, d'essence et de gazole obtenus.

Un autre avantage de la présente invention est de permettre une intégration thermique en utilisant un ou plusieurs fluides chauds disponibles dans le procédé tels que les produits de fond de la deuxième colonne, l'effluent inter réacteur et l'effluent du dernier réacteur de la section réactionnelle d'oligomérisation.

Un autre avantage de la présente invention est l'obtention de deux coupes essences, une coupe lourde riche en distillats moyens issue du fond de la colonne de préfractionnement à haute température et une coupe légère pauvre en distillats moyens issue du fond du débutaniseur à plus basse température qui permettent d'alimenter le séparateur à deux niveaux différents ce qui facilite la séparation d'une fraction essence et d'une fraction distillat moyen.

### DESCRIPTION DÉTAILLÉE DE L'INVENTION

Les réactions d'oligomérisation sont exothermiques, et la température de la section réactionnelle est généralement comprise entre 120°C et 300°C.

Conformément à l'invention un effluent produit par une section d'oligomérisation est envoyé dans une section de fractionnement qui comporte en premier lieu un préfractionnateur permettant de réaliser une étape de préfractionnement.

Au moins une partie de l'oligomérat issu de la section réactionnelle d'oligomérisation est donc envoyé, sans aucun traitement autre qu'un éventuel échange thermique via par exemple un échangeur de chaleur, dans une colonne de distillation dite préfractionnateur permettant un préfractionnement dudit oligomérat.

Ledit préfractionnement conduit à au moins deux fractions :
- une fraction de tête contenant un mélange gaz de pétrole liquéfié (GPL)/ essence légère, contenant moins de 15 % en poids par rapport à la charge d'hydrocarbures comprenant au moins 8 atomes de carbone, et préférentiellement moins de 10 %.
- une fraction de fond contenant un mélange essence lourde/ distillat moyen contenant moins de 15% en poids par rapport à la charge d'hydrocarbures comprenant au plus 5 atomes de carbone et préférentiellement moins de 8 %.

Selon une variante préférée de l'invention, le préfractionnateur comprend entre 5 et 20 plateaux théoriques et de préférence entre 8 et 18 plateaux théoriques et opère à une pression comprise entre 0,1 et 2 MPa, de préférence entre 0,3 et 1,2 MPa, la charge est alimentée sur un plateau situé en tête de la colonne, de préférence le plateau 1 à 6, les plateaux étant numérotés à partir de la tête de la colonne, les vapeurs de tête sont soutirées à une température comprise entre 100 et 150°C, de préférence comprise entre 110°C et 140°C, le liquide de fond est soutiré à une température comprise entre 170°C et 220°C, de préférence comprise entre 185°C et 205°C.

Conformément à l'invention, ladite fraction de tête est envoyée dans une seconde colonne dite débutaniseur qui conduit à au moins deux coupes :
- une coupe de tête contenant le GPL contenant les hydrocarbures comprenant 4 atomes de carbone ou moins,
- une coupe de fond contenant l'essence légère contenant les hydrocarbures comprenant au moins 5 atomes de carbone.

Selon une variante préférée, le débutaniseur comprend entre 15 et 35 plateaux théoriques et de préférence entre 18 et 30 plateaux théoriques et opère à la même pression que le préfractionateur, la charge est alimentée entre les plateaux théoriques 8 et 25 et de préférence entre les plateaux théoriques 10 et 18, les plateaux étant numérotés à partir de la tête de la colonne, les vapeurs de tête sont soutirées, condensées et sous refroidies à une température comprise entre 35 et 55°C de préférence comprise entre 40 et 50°C dans l'aérocondenseur, le liquide de fond est soutiré à une température comprise entre 100°C et 130°C, de préférence comprise entre 105 et 125°C.

Conformément à l'invention, ladite fraction de fond et au moins une partie de ladite coupe de fond sont envoyées dans une troisième colonne de distillation dite séparateur pour séparer les différents produits désirés les uns des autres. Les principaux produits obtenus sont de l'essence, et du distillat moyen (du kérosène et/ou du gasoil).

Selon une variante préférée, le séparateur comprend entre 20 et 40 plateaux théoriques et de préférence entre 25 et 35 plateaux théoriques et opère à une pression plus basse que la pression dudit débutaniseur comprise entre 0,01 MPa et 0,6 MPa et de préférence entre 0,05 et 0,3 MPa. Ladite fraction de fond issue du préfractionateur est alimentée entre les plateaux 5 et 15 et de préférence entre les plateaux 7 et 12, ladite coupe de fond issue du débutaniseur est alimentée entre les plateaux théoriques 2 et 12 et de préférence entre les plateaux 2 et 6, les plateaux étant numérotés à partir de la tête de la colonne. Les vapeurs de tête sont soutirées, condensées et sous refroidies à une température comprise entre 40°C et 80°C, de préférence comprise entre 50°C et 70°C dans l'aérocondenseur, le liquide de fond est soutiré à une température comprise entre 200°C et 240°C, de préférence comprise entre 210°C et 230°C.

De manière préférée, ladite fraction de fond et ladite coupe de fond alimentent le séparateur à deux niveaux différents, pour faciliter la séparation essence/distillat moyen. Le séparateur contient généralement entre 20 et 40 plateaux théoriques.

Dans le cas où ladite fraction de fond et ladite coupe de fond alimentent le séparateur à deux niveaux différents, ladite fraction de fond est introduite de préférence entre les plateaux théoriques 7 et 15 et ladite coupe de fond est introduite au-dessus, de préférence entre les plateaux 2 et 12, les plateaux étant numérotés à partir de la tête de la colonne.

Avantageusement, ladite coupe de fond est également en partie envoyée au pool essence, en partie purgée, en partie renvoyée dans la section réactionnelle d'oligomérisation ou de manière préférée en partie recyclée dans la colonne de préfractionnement.

Dans le cas où ladite coupe de fond est en partie recyclée dans la colonne de préfractionnement, ladite coupe de fond est préférentiellement introduite à quelques plateaux au-dessus du rebouilleur. De manière préférée, le taux de recyclage de ladite coupe de fond est compris entre 5 % en poids et 20 % en poids et de manière encore plus préférée entre 7 % en poids et 15 % en poids.

Sans augmenter la quantité d'énergie à apporter aux rebouilleurs, le préfractionnateur placé en amont du débutaniseur conduit à une réduction d'au moins 30 °C de la température de rebouillage nécessaire au fonctionnement du débutaniseur ce qui permet une intégration thermique en particulier grâce à un fluide chaud du procédé disponible à une température supérieure ou égale à 130°C ou grâce à une utilité chaude disponible à relativement bas niveau thermique. Par ailleurs, le recyclage partiel de ladite coupe de fond dans ladite colonne de préfractionnement conduit également à une réduction de la température de rebouillage dudit préfractionnateur comprise entre 5°C et 15°C.

Les différents fluides chauds du procédé permettent d'envisager plusieurs modes de mise en oeuvre de ladite intégration thermique.

Dans un premier mode de réalisation préféré, l'effluent de fond dudit séparateur est utilisé pour un échange thermique avec le rebouilleur dudit débutaniseur.

Dans un second mode de réalisation l'effluent issu du dernier réacteur est refroidi avant sa détente et son introduction dans le préfractionateur, ce qui permet de récupérer des calories et de réchauffer le rebouilleur dudit débutaniseur.

Ces deux modes de réalisation peuvent être combinés de façon à assurer une plus grande partie du rebouillage du débutaniseur via l'intégration thermique.

### DESCRIPTION DES FIGURES

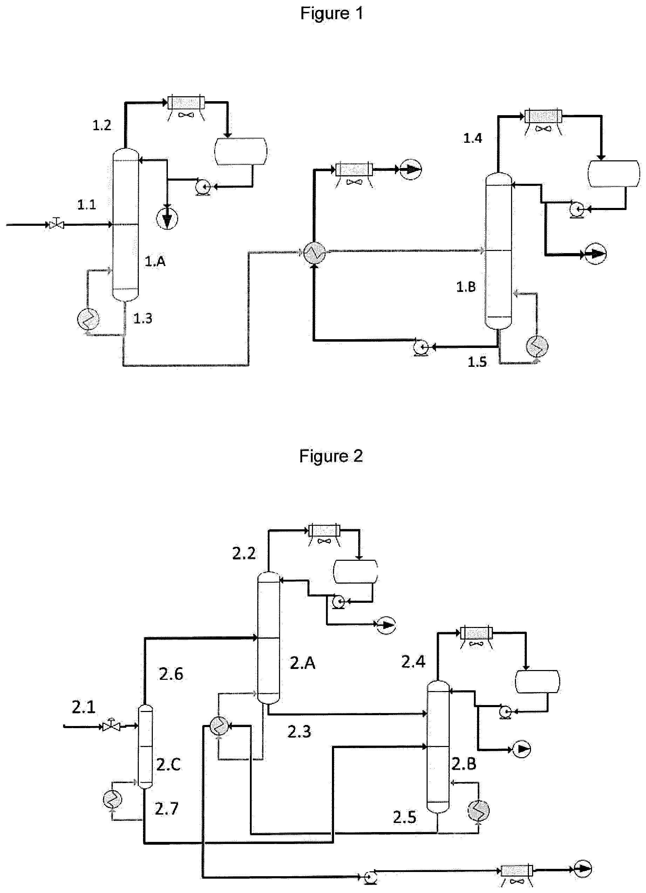

La figure 1 représente un schéma de procédé de fractionnement classique selon l'art antérieur pour obtenir une fraction GPL, une fraction essence et une fraction distillat moyen. L'oligomérat (1.1) est envoyé dans un débutaniseur (1.A) qui sépare au moins deux fractions, une en tête de colonne (1.2) et une en fond de colonne (1.3). Ladite fraction de fond de colonne (1.3) est ensuite envoyée dans un séparateur (1.B) qui sépare à son tour deux fractions (1.4 et 1.5).
La figure 2 représente un mode de réalisation du procédé selon l'invention dans lequel une colonne de préfractionnement (2.C) est installée en amont du procédé représenté à la figure 1. La fraction de tête contenant un mélange de gaz de pétrole liquéfié et d'essence légère (2.6) du préfractionnateur (2.C) est envoyée vers le débutaniseur (2.A) tandis que la fraction de fond contenant un mélange d'essence lourde et de distillat moyen (2.7) est envoyée directement dans le séparateur (2.B).

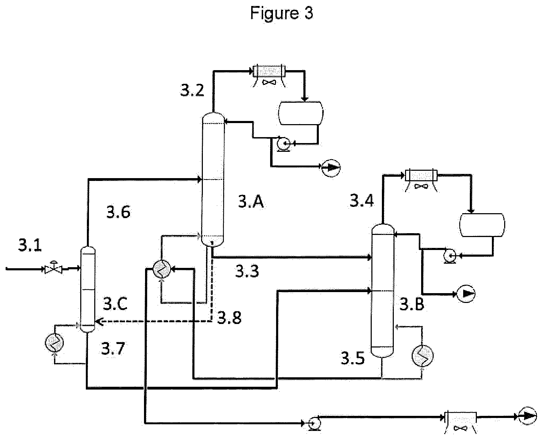

La figure 3 représente un mode de réalisation similaire à celui représenté à la figure 2 du procédé selon l'invention dans lequel, en outre une partie de la fraction de fond contenant un mélange d'essence lourde et de distillat moyen (3.7) du débutaniseur (3.A) est recyclé (3.8) dans ledit préfractionnateur (3.C).

### EXEMPLES

Dans l'ensemble des exemples 1 à 3, 21 t/h d'oligomérat issu d'une section d'oligomérisation composé de 28 % en poids d'hydrocarbures comprenant 4 atomes de carbone ou moins, 14 % en poids d'hydrocarbures comprenant 5 atomes de carbone, 10 % en poids d'hydrocarbures comprenant 6 atomes de carbone, 2 % en poids d'hydrocarbures comprenant 7 atomes de carbone, 12 % en poids d'hydrocarbures comprenant 8 atomes de carbone et 34 % en poids d'hydrocarbures comprenant au moins 9 atomes de carbone.

Les conditions opératoires sont ajustées de manière à fractionner ledit oligomérat en 3 produits dont les quantités sont résumées dans le tableau ci-dessous :

| Produit | Débit (t/h) |
|---|---|
| Gaz de Pétrole Liquéfié | 5,8 |
| Essence | 7,8 |
| Distillat Moyen | 7,4 |

Le gaz de pétrole liquéfié contient 2 % en poids d'hydrocarbures comprenant 5 atomes de carbone ou plus.

L'essence est caractérisée par un point d'ébullition initial D 86 de 13°C et un point d'ébullition final D 86 de 140°C et une teneur en d'hydrocarbures comprenant 4 atomes de carbone ou moins de seulement 1,9 % en poids.

Le distillat moyen est caractérisé par un point d'ébullition D 86 initial de 164°C et un point de flash méthode Nelson de 43,9°C.

### Exemple 1 (comparatif) :

Le procédé de l'exemple 1 est représenté à la figure 1. L'oligomérat (1.1) est envoyé dans un débutaniseur (1.A) qui sépare au moins deux fractions, une en tête de colonne (1.2) et une en fond de colonne (1.3). Ladite fraction de fond de colonne (1.3) est ensuite envoyée dans un séparateur (1.B) qui sépare à son tour deux fractions (1.4 et 1.5). Le produit de fond du débutaniseur (1.4) échange sa chaleur avec l'effluent l'alimentant (1.3). Le débutaniseur et le séparateur opèrent avec 23 plateaux théoriques les positions de leur alimentation sont optimisées de façon à minimiser les chaleurs dépensées aux rebouilleurs. Ainsi l'alimentation du débutaniseur (1.A) est placée au plateau 13 et l'alimentation du séparateur (1.B) est placée au plateau 8, les plateaux étant numérotés à partir de la tête de la colonne. Le débutaniseur (1.A) opère à 0,65 MPa et le séparateur (1.B) opère à 0,2 MPa.

### Exemple 2 (selon l'invention) :

Le procédé de l'exemple 2 est représenté à la figure 2. Dans l'exemple 2, une colonne de préfractionnement (2.C) est installée en amont du procédé de l'exemple 1. La fraction de tête du préfractionnateur (2.6) est envoyée dans le débutaniseur (2.A) tandis que la fraction de fond (2.7) est envoyée directement dans le séparateur (2.B).

Le préfractionnateur (2.C) comprend 14 plateaux théoriques et opère à 0,65 MPa. L'effluent de fond du séparateur (2.5) est utilisé pour un échange thermique avec le rebouilleur du débutaniseur (2.A) avant son refroidissement et stockage. La coupe de fond du débutaniseur (2.3) et la fraction de fond (2.7) issue du préfractionnement sont envoyées en totalité dans le séparateur (2.B) respectivement au plateau 4 et au plateau 10, les plateaux étant numérotés à partir de la tête de la colonne.

### Exemple 3 (selon invention):

Le procédé de l'exemple 3 est représenté à la figure 3. Dans l'exemple 3, le même procédé qu'à l'exemple 2 (figure 2) est mis en oeuvre avec en plus le recyclage (3.8) de 0,5 t/h de ladite coupe de fond (3.3) de le débutaniseur (3.A) dans le préfractionnateur (3.c) au niveau du plateau 12, les plateaux étant numérotés à partir de la tête de la colonne et la colonne contenant 14 plateaux.

| | Exemple 1 | Exemple 2 | Exemple 3 |
|---|---|---|---|
| **Préfractionateur** | | | |
| Débit de reflux (t/h) | | 11,9 | 11,9 |
| Service du rebouilleur (MW) | | 0,63 | 0,58 |
| Température du rebouilleur (°C) | | 197,6 | 187,1 |

| **Première colonne** | | | |
|---|---|---|---|
| Débit de reflux (t/h) | 13,9 | 12,8 | 12,8 |
| Service du rebouilleur latéral (MW) | | | |
| Température du rebouilleur latéral (°C) | | | |
| Service du rebouilleur (MW) | 1,0 | 0,53 | 0,52 |
| Température du rebouilleur (°C) | 145,0 | 116,7 | 116,4 |
| Service du condenseur (MW) | 1,9 | 1,75 | 1,75 |
| Température condenseur (°C) | 45,0 | 45,0 | 45,0 |

| **Deuxième colonne** | | | |
|---|---|---|---|
| Débit de reflux (t/h) | 5,4 | 4,2 | 4,1 |
| Service du rebouilleur (MW) | 1,20 | 1,08 | 1,13 |
| Température rebouilleur (°C) | 224 | 224 | 224 |
| Service du condenseur (MW) | 1,7 | 1,4 | 1,4 |
| Température condenseur (°C) | 45,0 | 61,0 | 61,0 |
| **Total Services rebouilleur** | **2,2** | **2,24** | **2,23** |
| **Total utilités chaudes (MW)** | **2,2** | **1,71** | **1,71** |
| | Non conforme | Conforme | Conforme |

Le procédé selon l'invention permet, pour les mêmes quantités et spécifications de produits obtenus, de diminuer la consommation des utilités chaudes de l'étape de fractionnement de l'oligomérat de 22 % par rapport à un fractionnement simple réalisé au moyen de 2 distillations successives sans section de préfractionnement.

De plus, par un simple recyclage partiel de la coupe essence légère pauvre en distillat moyen dans la colonne de préfractionnement, il est possible d'ajuster la proportion des coupes essence lourdes et légères, de façon à contrôler la température du rebouilleur de la colonne de préfractionnement.

## Revendications

1. Procédé d'oligomérisation d'oléfines légères dans lequel l'effluent de la section d'oligomérisation est envoyé dans un préfractionnateur qui conduit à au moins une fraction de tête contenant un mélange de gaz de pétrole liquéfié et d'essence légère et une fraction de fond contenant un mélange d'essence lourde et de distillat moyen, ladite fraction de tête étant envoyée dans un débutaniseur qui conduit à au moins une coupe gaz de pétrole liquéfié et une coupe essence légère, ladite fraction de fond et au moins une partie de ladite coupe essence légère étant envoyée dans un séparateur permettant d'obtenir au moins une fraction gazeuse, une fraction essence et une fraction gazole.

2. Procédé selon la revendication 1, dans lequel ladite fraction de tête contient un mélange gaz de pétrole liquéfié (GPL)/ essence légère, contenant moins de 15 % en poids par rapport à la charge d'hydrocarbures comprenant au moins 8 atomes de carbone, et ladite fraction de fond contient un mélange essence lourde/ distillat moyen contenant moins de 15% en poids par rapport à la charge d'hydrocarbures comprenant au plus 5 atomes de carbone.

3. Procédé selon les revendications 1 et 2, dans lequel ledit préfractionnateur comprend entre 5 et 20 plateaux théoriques, opère à une pression comprise entre 0,1 et 2 MPa, la charge étant alimentée sur un plateau situé en tête de la colonne, les vapeurs de tête étant soutirées à une température comprise entre 100 et 150°C et le liquide de fond étant soutiré à une température comprise entre 170°C et 220°C.

4. Procédé selon l'une quelconque des revendications précédentes, dans lequel ladite coupe gaz de pétrole liquéfié contient les hydrocarbures comprenant 4 atomes de carbone ou moins et ladite coupe essence légère contient les hydrocarbures comprenant au moins 5 atomes de carbone.

5. Procédé selon l'une quelconque des revendications précédentes, dans lequel ledit débutaniseur comprend entre 15 et 35 plateaux théoriques et opère à la même pression que le préfractionateur, la charge étant alimentée entre les plateaux théoriques 8 et 25, les plateaux étant numérotés à partir de la tête de la colonne, les vapeurs de tête étant soutirées, condensées et sous refroidies à une température comprise entre 35 et 55°C dans un aérocondenseur, le liquide de fond étant soutiré à une température comprise entre 100°C et 130°C.

6. Procédé selon l'une quelconque des revendications précédentes, dans lequel ledit séparateur comprend entre 20 et 40 plateaux théoriques et opère à une pression plus basse que la pression dudit débutaniseur comprise entre 0,01 MPa et 0,6 MPa.

7. Procédé selon l'une quelconque des revendications précédentes, dans lequel ladite fraction de fond issue du préfractionateur est alimentée entre les plateaux 5 et 15 dudit séparateur, ladite coupe de fond issue du débutaniseur est alimentée entre les plateaux théoriques 2 et 12 dudit séparateur, les plateaux étant numérotés à partir de la tête de la colonne, les vapeurs de tête étant soutirées, condensées et sous refroidies à une température comprise entre 40°C et 80°C dans un aérocondenseur et le liquide de fond étant soutiré à une température comprise entre 200°C et 240°C.

8. Procédé selon l'une quelconque des revendications précédentes, dans lequel ladite fraction de fond et ladite coupe de fond alimentent ledit séparateur à deux niveaux différents.

9. Procédé selon l'une quelconque des revendications précédentes, dans lequel ladite coupe de fond est en partie recyclée dans la colonne de préfractionnement.

10. Procédé selon la revendication 9, dans lequel ladite coupe de fond est préférentiellement introduite à quelques plateaux au-dessus du rebouilleur.

11. Procédé selon les revendications 9 et 10, dans lequel le taux de recyclage de ladite coupe de fond est compris entre 5 % en poids et 20 % en poids.

12. Procédé selon l'une quelconque des revendications précédentes, dans lequel l'effluent de fond dudit séparateur est utilisé pour un échange thermique avec le rebouilleur dudit débutaniseur.

13. Procédé selon l'une quelconque des revendications précédentes, dans lequel l'effluent issu du dernier réacteur est refroidi avant sa détente et son introduction dans le préfractionateur, ce qui permet de récupérer des calories et de réchauffer le rebouilleur dudit débutaniseur.

## Patentansprüche

1. Verfahren zur Oligomerisation von Leichtolefinen, wobei der Abstrom aus dem Oligomerisationsabschnitt zu einem Vorfraktionierer geleitet wird, der zu mindestens einer Kopffraktion, die eine Mischung aus Flüssiggas und Leichtbenzin enthält, und einer Sumpffraktion führt, die eine Mischung aus Schwerbenzin und Mitteldestillat enthält, wobei die Kopffraktion zu einem Debutanisierer geleitet wird, der zu mindestens einem Flüssiggasschnitt und einem Leichtbenzinschnitt führt, wobei die Sumpffraktion und mindestens ein Teil des Leichtbenzinschnitts zu einem Abscheider geleitet werden, der es ermöglicht, eine gasförmige Fraktion, eine Benzinfraktion und eine Gasölfraktion, zu erhalten.

2. Verfahren nach Anspruch 1, wobei die Kopffraktion eine Mischung aus Flüssiggas (LPG) und Leichtbenzin enthält, die weniger als 15 Gew.-%, bezogen auf die Kohlenwasserstoff-Charge, die mindestens 8 Kohlenstoffatome aufweist, und die Sumpffraktion eine Schwerbenzin/Mitteldestillat-Mischung enthält, die weniger als 15 Gew.-%, bezogen auf die Kohlenwasserstoff-Charge, das höchstens 5 Kohlenstoffatome aufweist, enthält.

3. Verfahren nach den Ansprüchen 1 und 2, wobei der Vorfraktionierer zwischen 5 und 20 theoretische Böden enthält, bei einem Druck im Bereich zwischen 0,1 und 2 MPa arbeitet, wobei die Charge einem Boden zugeführt wird, der sich am Kopf der Kolonne befindet, wobei die Kopfdämpfe bei einer Temperatur im Bereich zwischen 100 und 150 °C abgezogen werden, und die Sumpfflüssigkeit bei einer Temperatur im Bereich zwischen 170 °C und 220 °C abgezogen wird.

4. Verfahren nach einem der vorhergehenden Ansprüche, wobei der Flüssiggasschnitt Kohlenwasserstoffe enthält, die 4 oder weniger Kohlenstoffatome aufweisen und wobei der Leichtbenzinschnitt die Kohlenwasserstoffe enthält, die mindestens 5 Kohlenstoffatome aufweisen.

5. Verfahren nach einem der vorhergehenden Ansprüche, wobei der Debutanisierer zwischen 15 und 35 theoretische Böden aufweist und beim gleichen Druck arbeitet wie der Vorfraktionierer, wobei die Charge zwischen den theoretischen Böden 8 und 25 zugeführt wird, wobei die Böden ausgehend vom Kolonnenkopf nummeriert sind, wobei die Kopfdämpfe abgezogen, kondensiert und bei einer Temperatur im Bereich zwischen 35 und 55 °C in einem luftgekühlten Kondensator unterkühlt werden, wobei die Sumpfflüssigkeit bei einer Temperatur im Bereich zwischen 100 °C und 130 °C abgezogen wird.

6. Verfahren nach einem der vorhergehenden Ansprüche, wobei der Abscheider zwischen 20 und 40 theoretische Böden enthält und bei einem niedrigeren Druck als dem Druck des Debutanisieres im Bereich zwischen 0,01 MPa und 0,6 MPa arbeitet.

7. Verfahren nach einem der vorhergehenden Ansprüche, wobei die aus dem Vorfraktionierer erhaltene Sumpffraktion zwischen den Böden 5 und 15 des Abscheiders zugeführt wird, wobei die aus dem Debutanisierer erhaltene Sumpffraktion zwischen den theoretischen Böden 2 und 12 des Abscheiders zugeführt wird, wobei die Böden ausgehend vom Kolonnenkopf nummeriert sind, wobei die Kopfdämpfe abgezogen, kondensiert und bei einer Temperatur im Bereich zwischen 40 °C und 80 °C in einem luftgekühlten Kondensator unterkühlt werden, und wobei die Sumpfflüssigkeit bei einer Temperatur im Bereich zwischen 200 °C und 240 °C abgezogen wird.

8. Verfahren nach einem der vorhergehenden Ansprüche, wobei die Sumpffraktion und der Sumpfschnitt dem Abscheider in zwei verschiedenen Höhen zugeführt werden.

9. Verfahren nach einem der vorhergehenden Ansprüche, wobei der Sumpfschnitt teilweise in die Vorfraktionierungskolonne zurückgeführt wird.

10. Verfahren nach Anspruch 9, wobei der Sumpfschnitt vorzugsweise in mehrere Böden über dem Rückverdampfer eingeführt wird.

11. Verfahren nach den Ansprüchen 9 und 10, wobei der Anteil der Rückführung des Sumpfschnitts im Bereich zwischen 5 Gew.-% und 20 Gew.-% liegt.

12. Verfahren nach einem der vorhergehenden Ansprüche, wobei der Sumpfabstrom des Abscheiders zum Wärmeaustausch mit dem Rückverdampfer des Debutanisierer verwendet wird.

13. Verfahren nach einem der vorhergehenden Ansprüche, wobei der aus dem letzten Reaktor erhaltene Abstrom vor der Druckentlastung und der Einführung in den Vorfraktionierer gekühlt wird, wodurch es ermöglicht wird, Wärme wiederzugewinnen und den Rückverdampfer des Debutanisierers wieder zu erwärmen.

## Claims

1. Process for the oligomerisation of light olefins in which the effluent from the oligomerisation section is passed to a prefractionator, which leads to at least one head fraction containing a mixture of liquefied petroleum gas and light gasoline and a bottom fraction containing a mixture of heavy gasoline and middle distillate, the said head fraction being passed to a debutaniser that leads to at least one liquefied petroleum gas cut and a light gasoline cut, the said bottom fraction and at least part of the said light gasoline cut being passed to a separator enabling at least a gaseous fraction, a gasoline fraction and a gasoil fraction to be obtained.

2. Process according to Claim 1, in which the said head fraction contains a liquefied petroleum gas (LPG) / light gasoline mixture, containing less than 15% by weight with respect to the hydrocarbon feedstock containing at least 8 carbon atoms, and the said bottom fraction containing a heavy gasoline / middle distillate mixture containing less than 15% by weight with respect to the hydrocarbon feedstock containing at most 5 carbon atoms.

3. Process according to Claims 1 and 2, in which the said prefractionator contains between 5 and 20 theoretical trays, operates at a pressure between 0.1 and 2 MPa, the feedstock being supplied to a tray situated at the head of the column, the head vapours being withdrawn at a temperature between 100° and 150°C and the bottom liquid being withdrawn at a temperature between 170°C and 220°C.

4. Process according to any one of the preceding claims, in which the said liquefied petroleum gas cut contains hydrocarbons containing 4 or fewer carbon atoms and the said light gasoline cut contains hydrocarbons containing at least 5 carbon atoms.

5. Process according to any one of the preceding claims, in which the said debutaniser contains between 15 and 35 theoretical trays and operates at the same pressure as the prefractionator, the feedstock being fed between the theoretical trays 8 and 25, the trays being numbered starting from the head of the column, the head vapours are removed, condensed and subcooled to a temperature between 35° and 55°C in an air-cooled condenser, and the bottom liquid is removed at a temperature between 100°C and 130°C.

6. Process according to any one of the preceding claims, in which the said separator contains between 20 and 40 theoretical trays and operates at a lower pressure than the pressure of the said debutaniser, namely between 0.01 MPa and 0.6 MPa.

7. Process according to any one of the preceding claims, in which the said bottom fraction obtained from the prefractionator is fed between the trays 5 and 15 of the said separator, the said bottom fraction obtained from the debutaniser is fed between the theoretical trays 2 and 12 of the said separator, the trays being numbered starting from the head of the column, the head vapours being removed, condensed and subcooled to a temperature between 40°C and 80°C in an air-cooled condenser and the bottom liquid being removed at a temperature between 200°C and 240°C.

8. Process according to any one of the preceding claims, in which the said bottom fraction and the said bottom cut supply the said separator at two different levels.

9. Process according to any one of the preceding claims, in which the said bottom cut is in part recycled to the prefractionation column.

10. Process according to Claim 9, in which the said bottom cut is preferably introduced to several trays above the reboiler.

11. Process according to Claims 9 and 10, in which the degree of recycling of the said bottom cut is between 5% by weight and 20% by weight.

12. Process according to any one of the preceding claims, in which the bottom effluent from the said separator is used for a heat exchange with the reboiler of the said debutaniser.

13. Process according to any one of the preceding claims, in which the effluent obtained from the last reactor is cooled before its pressure is released and it is introduced to the prefractionator, which enables heat to be recovered and the reboiler of the said debutaniser to be reheated.
